(19) Europäisches Patentamt

European Patent Office

Office européen des brevets

(11) Veröffentlichungsnummer: **0 276 772 B1**

(12) **EUROPÄISCHE PATENTSCHRIFT**

(45) Veröffentlichungstag der Patentschrift: **15.07.92**

(21) Anmeldenummer: **88100895.7**

(22) Anmeldetag: **22.01.88**

(51) Int. Cl.⁵: **C11B 15/00, A61K 31/23**

(54) **Verfahren zur Herstellung von mikrodispersen Fischölpräparaten.**

(30) Priorität: **24.01.87 DE 3702031**

(43) Veröffentlichungstag der Anmeldung:
**03.08.88 Patentblatt 88/31**

(45) Bekanntmachung des Hinweises auf die
Patenterteilung:
**15.07.92 Patentblatt 92/29**

(84) Benannte Vertragsstaaten:
**AT BE CH DE ES FR GB GR IT LI NL SE**

(56) Entgegenhaltungen:
**US-A- 4 447 418**

**PATENT ABSTRACTS OF JAPAN, unexamined applications, Sektion C, Band 7, Nr. 171,
28. Juli 1983 THE PATENT OFFICE JAPANESE
GOVERNMENT Seite 149 C 178, Kokai-Nr.
58-79 912 (SHISEIDO K.K.)**

**PATENT ABSTRACTS OF JAPAN, unexamined applications, Sektion C, Band 9, Nr. 228,
13. September 1985 THE PATENT OFFICE JA-
PANESE GOVERNMENT Seite 33 C 303,
Kokai-Nr. 60-87 205 (SHISEIDO K.K.)**

(73) Patentinhaber: **BASF Aktiengesellschaft
Carl-Bosch-Strasse 38
W-6700 Ludwigshafen(DE)**

(72) Erfinder: **Horn, Dieter, Dr.
Schroederstrasse 69
W-6900 Heidelberg(DE)**
Erfinder: **Ouadbeck-Seeger, Hans-Juergen,
Prof. Dr.
Heinrich-Baermann-Strasse 5
W-6702 Bad Duerkheim(DE)**
Erfinder: **Frickel, Fritz-Frieder, Dr.
Silvanerweg 7
W-6705 Deidesheim(DE)**

**Beschreibung**

Die Erfindung betrifft ein Verfahren zur Überführung von Fischöl in eine wäßrige oder pulverförmige, wasserlösliche Form, die als weitgehend geschmacks- und geruchsneutrales orales Prophylaktikum gegen die Entwicklung atherosklerotischer Veränderungen eingesetzt werden kann.

Epidemiologische Studien unter Eskimo-Populationen haben gezeigt, daß signifikant niedrigere Blutfettwerte sowie eine signifikant niedrigere Inzidenz von Bluthochdruck im Vergleich zu Populationen westlicher Industrienationen auf den erhöhten Verzehr von Fischölen zurückgeführt werden kann (M.H. Davidson, Ph.R. Liebson, Cardiovascular Reviews & Reports 7, 461 -472 (1986)).

Als prophylaktisch aktive Substanzen werden insbesondere Eikosapentaensäure (EPA) sowie Dokosahexaensäure (DHA) angesehen.

Nach dem Stand der Technik werden Fischöle mit einem hohen Gehalt an EPA und DHA in Form von Weichgelatinekapseln oral verabreicht. Bei einer Dosierung von 500 - 1000 mg Öl pro Kapsel handelt es sich um eine voluminöse, für den Patienten wenig akzeptable Verabreichungsform. Nach Auflösung der Gelatinewand während der Passage im Magen-Darm-Trakt wird das Öl in Form eines makroskopischen Tropfens freigesetzt, was die rasche Resorption beeinträchigt, die bekanntlich eine möglichst feinteilige Emulsionsform voraussetzt. Einen gewissen Fortschritt demgegenüber bringen technologische Maßnahmen mit dem Ziel, Fischöle in Mikrokapseln zu portionieren (Jpn. Kokai Tokkyo Koho JP 61 15,733 [86 15, 733], 23.01.86), um in erster Linie den unangenehmen Geschmack und Geruch der Fischöle zu maskieren. Die Teilchengrößen liegen bei dieser Verfahrensweise allerdings immer noch im makroskopischen Bereich bei einem Durchmesser von 80 $\mu$m und einem Ölgehalt von 45 %. Dementsprechend sind sie schlecht dispergierbar und liefern nur grobdisperse, rasch sedimentierende Suspensionen, die für eine orale Applikation wenig geeignet sind.

Es bestand die Aufgabe, ein Verfahren aufzuzeigen, das Präparate von Fischölen liefert, die die genannten Nachteile nicht aufweisen, bei denen die Ölkomponente möglichst hochkonzentriert in extrem feinteiliger und leicht dispergierbarer, kolloidstabiler Form in einer flüssigen oder pulverförmigen Matrix vorliegt, und bei denen trotz der Feinteiligkeit der unangenehme Geschmack und Geruch unterdrückt wird.

Diese Aufgabe wird erfindungsgemäß gelöst durch ein Verfahren nach Anspruch 1, vorzugsweise nach Anspruch 2.

Das Verfahren nach Anspruch 1 erfordert eine Ein- oder Mehrfachemulgierung mit Hilfe eines herkömmlichen Schnellrührers, z.B. eines Ultra Turrax®. Damit werden jedoch auch bei mehrfacher Passage bzw. längerer Einwirkungszeit nur relativ grobe Emulsionen erhalten. Die so voremulgierte Mischung wird in einem modernen Hochdruckhomogenisator, beispielsweise Microfluidizer® der Fa. Biotechnology Development Corp., Newton, Mass., USA, zu einer fertigen O/W-Emulsion verarbeitet. Lösliche Zusätze, insbesondere z.B. Oxidationsstabilisatoren, werden zweckmäßig von vornherein, d.h. noch vor dem Voremulgieren, zugesetzt, unlösliche Zusätze werden gegebenenfalls erst der fertigen Emulsion zugemischt. Man erhält auf diese Weise Emulsionen mit mittleren Öltröpfchendurchmessern unter 10, vorzugsweise unter 2 $\mu$m. Die bevorzugten Emulsionen enthalten praktisch keine Anteile mit Durchmessern über 4 $\mu$m. Anschließend kann das Wasser nach üblichen Methoden entzogen werden.

Bei dem Verfahren nach Anspruch 2 wird das Fischöl in einem flüchtigen, mit Wasser mischbaren organischen Lösungsmittel, gemeinsam mit einem Tensid und ggf. weiteren galenischen Hilfsstoffen, bei Temperaturen zwischen 10 und 200°C, vorzugsweise 100 und 180°C, gegebenenfalls unter erhöhtem Druck, bei Anwendung von Temperaturen an der Obergrenze des genannten Bereichs zweckmäßig innterhalb einer Zeit von weniger als 10 Sekunden gelöst, die erhaltene moleculardisperse Lösung durch Mischen mit einer wäßrigen Lösung eines Schutzkolloids bei Temperaturen zwischen 0 und 50°C in eine Zweiphasenmischung zerlegt, wobei die Ölphase in Form emulgierter submikroskopisch kleiner Teilchen (mittlere Teilchengröße von weniger als 1, vorzugsweise unter 0,5 $\mu$m) entsteht, das erhaltene Zweiphasengemisch in an sich bekannter Weise von dem Lösungsmittel befreit und ggf. durch gleichzeitigen oder nachfolgenden Entzug von Wasser in ein Trockenpulver überführt. Besonders bevorzugt werden Emulsionen, die praktisch keine Fischöltröpfchen mit Durchmessern über 1 $\mu$m enthalten.

Derartige Präparate sind generell zur Unterstützung der Ernährung interessant und können in flüssiger oder Pulverform oder tablettiert oral appliziert werden.

Die bevorzugte Arbeitsweise gemäß Anspruch 2 macht sich den Umstand zunutze, daß die in der Kälte meist geringe Löslichkeit der Fischöle in den wassermischbaren Lösungsmitteln mit steigender Temperatur deutlich zunimmt. Doch bestand gegen das Erhitzen ein starkes Vorurteil, da insbesondere die stark ungesättigten Öle als thermisch labil gelten und eine die physiologische Wirkung nachteilig beeinflussende Isomerisierung oder thermische Zerstörung erwartet wurde. Ein weiteres Vorurteil gegen die Herstellung extrem feinteiliger Mikronisate bestand darin, daß durch das starke Ansteigen der spezifischen Oberfläche mit abnehmender Teilchengröße die Stabilität der oxidationsempfindlichen Fischöle nachteilig beeinflußt und weiterhin der unangenehme Geschmack und unangenehme Geruch insbesondere der Fischöle verstärkt und somit die diätetische Verwendung solcher Präparate beeinträchtigt würde.

Es ist daher überraschend, daß die erfindungsgemäß hergestellten Präparate außerordentlich stabil, weitgehend geschmacksneutral und nahezu geruchlos sind. Die erfindungsgemäße Vorgehensweise ermöglicht darüber hinaus auf einfache Weise eine Aromatisierung der Präparate durch Zugabe geeigneter Aromaträger über die Lösungsmittel-oder Schutzkolloidphase.

Durch entsprechende Wahl des Schutzkolloids können Pulverpräparate hergestellt werden, die in ihrem Auflösungsverhalten in wäßrigen Medien beliebig zwischen rasch kaltwasserlöslich bis zu schwerlöslich eingestellt werden können, wobei jeweils die Ölphase in Form submikroskopisch kleiner, leicht resorbierbarer Teilchen vorliegt. Es ist sogar möglich, Präparate herzustellen, die die mikrodisperse Ölphase erst nach enzymatischem Abbau des Schutzkolloids und pH-gesteuert während der Darm-Passage freisetzen. Dies ist wichtig, um zu vermeiden, daß der Fischgeschmack beim Aufstoßen doch noch unangenehm empfunden wird.

Diese Formulierungen besitzen die Eigenschaften eines Depotpräparates. Keines der bekannten Verfahren liefert Präparate, bei denen durch Wahl des Schutzkolloids die Freigabe der Ölphase variabel gestaltet und gezielt gesteuert werden kann.

Als Fischöl werden die an mehrfach ungesättigten Fettsäuren (insbesondere EPA und DHA) reichen Öle von Kaltwasserfischen bevorzugt. Neben den natürlichen Fischölen können auch durch Umesterung modifizierte, an EPA und DHA angereicherte Fischöle eingesetzt werden.

Die Matrix dieser Präparate wird im Fall der Pulverpräparate gebildet aus dem homogen verteilten Schutzkolloid, Tensid, gegebenenfalls Weichmacher, Stabilisatoren u.a. galenischen Hilfsstoffen. Im Fall der wäßrigen Präparate ist es die wäßrige Lösung dieser Stoffe.

Zur Durchführung des bevorzugten Verfahrens nach Anspruch 2 sind vor allem wassermischbare, thermisch stabile, flüchtige, nur Kohlenstoff, Wasserstoff und Sauerstoff enthaltende Lösungsmittel wie Alkohole, Ether, Ester, Ketone und Acetale geeignet. Vorzugsweise werden Methanol, Ethanol, n-Propanol, Isopropanol, 1,2-Butandiol-1-methylether, 1,2-Propandiol-1-n-propylether, Tetrahydrofuran, Dioxan, Ethylmethylether, Methylacetat oder Aceton verwendet. Allgemein verwendet man zweckmäßig solche Lösungsmittel, die mindestens zu 10 % wassermischbar sind, einen Siedepunkt unter 200°C aufweisen und weniger als 7 Kohlenstoffatome haben.

Als Schutzkolloide werden beispielsweise Polypeptide wie Gelatine, Kasein, Kaseinat, Polysaccharide wie Stärke, Dextrin, Dextran, Pektin, Gummiarabicum, ferner Milch, Milchpulver oder Mischungen davon verwendet. Es können aber auch Polyvinylalkohol, Polyvinylpyrrolidon, Vinylpyrrolidon-Vinylacetat-Copolymere, Acrylsäure- und Methacrylsäurecopolymere mit Acrylsäure- bzw. Methacrylsäureestern, Methylcelluose, Carboxymethylcellulose, Hydroxypropylcellulose und Alginate eingesetzt werden. Besonders vorteilhaft ist, wenn - wie beispielsweise bei Milch, Kasein, (Meth-)Acrylsäure-Polymerisation, - die Wasserlöslichkeit des Schutzkolloids über den pH-Wert eingestellt werden kann. Bezüglich näherer Einzelheiten wird auf R.A. Morton, Fast Soluble Vitamins, Intern. Encyclopedia of Food and Nutrition, Bd. 9, Pergamon Press 1970, S. 128 -131, verwiesen. Zur Erhöhung der mechanischen und oxidativen Stabilität des pulverförmigen Endproduktes (z.B. Vermeidung von Staubbildung) ist es zweckmäßig, dem Kolloid einen Weichmacher zuzusetzen wie Zucker oder Zuckeralkohole, z.B. Saccharose, Glucose, Lactose, Invertzucker, Sorbit, Mannit oder Glycerin.

Zur Erhöhung der Stabilität des Fischöls gegen oxidativen Abbau ist es ferner vorteilhaft, Stabilisatoren wie α-Tocopherol, t-Butyl-hydroxytoluol, t-Butylhydroxyanisol oder Ethoxyquine zuzusetzen, und zwar in Mengen von bis zu 20, vorzugsweise 2 bis 10 Gew.%, bezogen auf Fischöl. Sie können entweder der wäßrigen oder der Lösungsmittel-Phase zugesetzt werden, vorzugsweise werden sie jedoch gemeinsam mit dem Fischöl und einem Tensid in der Lösungsmittelphase gelöst.

Als Tenside geeignet sind z.B. Ester langkettiger Fettsäure mit Ascorbinsäure, Fettsäuremono- und diglyceride und deren Oxethylierungsprodukte, Ester von Monofettsäureglyceriden mit Essigsäure, Zitronensäure, Milchsäure oder Diacetylweinsäure, 2-(2′-stearoyllactyl)milchsaure Salze, Polyglycerinfettsäureester (z.B. das Monostearat von Triglycerin), Sorbitanfettsäureester, Propylenglykol-Fettsäureester und Lecithin. Besonders bevorzugt wird Ascorbylpalmitat.

Das Verhältnis Tensid, Schutzkolloid und Weichmacher zu Öl wird im allgemeinen so gewählt, daß ein Endprodukt erhalten wird, das 5 bis 70 Gew.%, vorzugsweise 50 - 60 Gew.% Fischöl enthält, sowie 1 bis 30, vorzugsweise 5 bis 15 Gew.% eines oder mehrerer Tenside, 5 - 50, vorzugsweise 10 bis 40 Gew.% eines Schutzkolloids, 0 - 70, vorzugsweise 3 bis 35 Gew.% eines Weichmachers, ferner je 0 bis 10 Gew.%, insgesamt maximal 50 Gew.%, alle Prozentangaben bezogen auf Trockenmasse, eines Aromastoffs und eines Stabilisators und weiterer üblicher galenischer Hilfsstoffe, wobei die wäßrige Zubereitung bzw. das Pulver die Fischölkomponente in Form kleinster Teilchen bei einer mittleren Teilchengröße von weniger als 10, vorzugsweise weniger als 1, insbesondere weniger als 0,5 $\mu$m enthält und praktisch keinen Anteil mit einer Korngröße über 20, vorzugsweise über 4 $\mu$m (bei dem Verfahren nach Anspruch 1) bzw. keine über 2, vorzugsweise keine über 1 $\mu$m (bei dem Verfahren nach Anspruch 2) aufweist.

Zur Einfärbung der Präparate können Lebensmittelfarbstoffe wie Carotinoide der Lösungsmittelphase zugesetzt werden. Zur Vitaminisierung der Präparate werden hydrophobe Vitamine dem Fischöl bzw. (im Falle des Verfahrens nach Anspruch 2) der Lösungsmittelphase und hydrophile Vitamine der wäßrigen Schutzkolloidphase zugesetzt. Als weitere übliche galenische Hilfsmittel kommen neben den bereits genannten Weichmachern, Aromastoffen und Stabilisatoren beispielsweise folgende in Betracht: Spreng-, Binde-, Netzmittel, Farbstoffe, Konservierungsmittel, den pH-Wert beeinflussende Zusätze (vgl. H. Sucker et al. Pharmazeutische Technologie, Thieme-Verlag, Stuttgart 1978).

Das erfindungsgemäße Verfahren nach Anspruch 1 oder 2 liefert zunächst je nach Art und Menge des verwendeten Schutzkolloids eine viskose Flüssigkeit, die im Falle eines gelierfähigen Kolloids gelartig erstarrt. Diese Flüssigkeit kann als solche oral verabreicht werden.

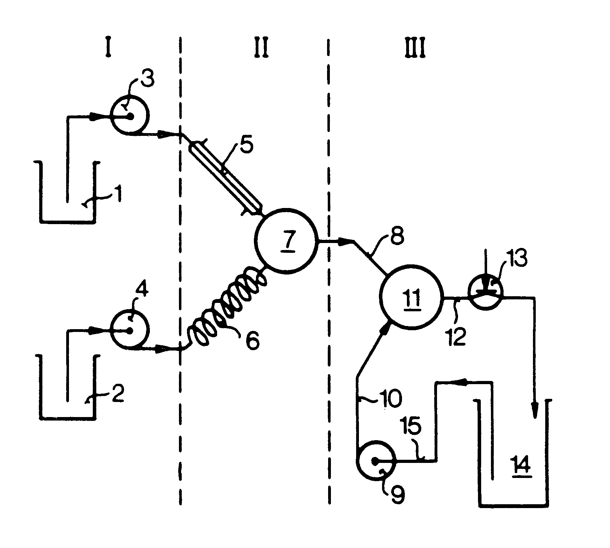

Im einzelnen führt man das bevorzugte Verfahren nach Anspruch 2 beispielsweise mit einer Apparatur, wie sie in Fig. 1 schematisch dargestellt ist, wie folgt durch:

Die Apparatur gliedert sich in die Teile I, II und III. Teil II ist gegebenenfalls der Hochtemperaturabschnitt, während in den Teilen I und III die Temperaturen weniger als 50°C betragen.

Im Gefäß (1) wird eine Lösung des Öls in dem ausgewählten Lösungsmittel in Konzentrationen von 2 - 40 Gew.%, bezogen auf die Mischung, gegebenenfalls unter Zusatz von je 0,1 - 10 Gew.-% an Stabilisatoren und Tensiden, vorgelegt. Gefäß (2) enthält das Lösungsmittel ohne Beimischung des Öls. Über die Pumpen (3) bzw. (4) werden die Fischöllösung und das Lösungsmittel der Mischkammer (7) zugeführt, wobei das Mischungsverhältnis durch Wahl der jeweiligen Förderleistung der Pumpen vorgegeben werden kann und so gewählt wird, daß je nach Lösungsmittel und Verweilzeit eine Öl-Konzentration in der Mischkammer von 0,5 - 10 Gew.-%, bezogen auf die Lösung, entsteht. Das Mischkammervolumen (7) ist so bemessen, daß bei der gewählten Förderleistung der Pumpen (3) und (4) die Verweilzeit in (7) vorzugsweise weniger als 1 Sekunde beträgt, wenn das Fischöl bei der gewählten Temperatur nicht thermisch und oxidativ stabil ist.

Das Lösungsmittel wird vor Eintritt in die Mischkammer über den Wärmetauscher (6) auf die gewünschte Temperatur gebrachte, während die Fischöllösung durch Zuführung über die thermisch isolierte Zuleitung (5) bei Temperauren unterhalb 50°C gehalten wird. Durch turbulente Mischung in (7) erfolgt im Temperaturbereich 10-200°C, vor allem 80 bis 180°C, insbesondere bei 110 - 160°C, die Lösung des Fischöls, und die erhaltene Lösung tritt über (8) nach kurzer Verweilzeit, vorzugsweise von weniger als 1 Sekunde, in die zweite Mischkammer (11) ein, in der durch Zumischen von Wasser oder einer wäßrigen Schutzkolloidlösung über die Pumpe (9) und die Zuleitung (10) die Phasentrennung der Ölphase in kolloiddisperser Form erfolgt. Über Leitung (12) wird sodann das feinteilige Zweiphasengemisch über das Überdruckventil (13) ausgetragen und dem Vorratsgefäß (14) zugeführt. Zur Erzielung einer möglichst hohen Wirkstoffkonzentration kann das Zweiphasengemisch über die Saugleitung (15) im Kreis geführt werden.

Bei Einstellung des Überdruckventil (13) auf Drücke oberhalb 1 bar können in dem neuen Verfahren sogar Lösungsmittel bei Temperaturen oberhalb ihres Siedepunktes (bei Normaldruck) verwendet werden.

Aus der Emulsion kann eine pulverförmige Präparat in an sich bekannter Weise, z.B. durch Sprühtrocknen oder durch Sprühkühlen und Einhüllen der Teilchen, Abtrennen und Trocknen im Wirbelbett erfolgen.

Zum Sprühtrocknen wird die Emulsion gegebenenfalls entweder zuerst vom Lösungsmittel durch Destillation, vorzugsweise unter vermindertem Druck, oder durch Extraktion mit einem mit Wasser nicht mischbaren Lösungsmittel befreit, oder die gesamte Mischung wird sprühgetrocknet und so Wasser und Lösungsmittel zusammen im Sprühturm abgezogen.

Am Boden des Sprühturms fällt das Präparat meist bereits trocken und rieselfähig an. In manchen Fällen kann es zweckmäßig sein, die vollständige Trocknung zwischen in einem Wirbelbett vorzunehmen.

Anstelle der Herstellung der Pulverzubereitung durch Sprühtrocknen können auch beliebige andere Methoden angewendet werden, um die in dem Zweiphasengemisch bereits feinverteilten Ölteilchen in die Pulverform zu überführen.

Ein bekanntes und hier gleichermaßen anwendbares Verfahren besteht z.B. darin, die vom Lösungsmittel befreite Emulsion mit Paraffinöl zu emulgieren, die Mischung abzukühlen, das Paraffinöl von den gelierten Schutzkolloidteilchen zu trennen, das erhaltene Präparat mit Benzin zu waschen und im Wirbelbett zu trocknen.

Man erhält ein Trockenpulver, das bei Verwendung eines wasserlöslichen Kolloids erneut in Wasser unter Erzielung einer gleichmäßigen Feinverteilung des Fischöls im Korngrößenbereich < 10, vorzugsweise

< 1 μm gelöst werden kann. Im Stabilitätstest erweist sich das so erhaltene Wirkstoff-Hydrosol trotz der Feinverteilung als außerordentlich stabil.

Bei der bevorzugten Arbeitsweise nach Anspruch 1 ist es besonders überraschend, daß beim Mischen der Öllösung mit der wäßrigen Lösung des Schutzkolloids ein außerordentlich feinteiliges und dennoch kolloid- und oxidationsstabiles Ölpräparat erhalten wird. Es ist weiterhin überraschend, daß der hohe Feinverteilungsgrad auch während der Aufarbeitung und Überführung in die Pulverform erhalten bleibt und sogar nach Wiederauflösung des Pulverpräparates in einem wäßrigen Medium unverändert vorliegt. Es ist so ohne Schwierigkeit möglich, Präparate zu erhalten, in denen der Hauptanteil der Ölphase in einer Teilchengröße von 0,2 - 0,3 μm vorliegt. Bei der Arbeitsweise nach Anspruch 1 ohne Lösungsmittel wird das Fischöl meist nicht ganz so extrem fein verteilt, aber auch hier können durchschnittliche Teilchengrößen (Tröpfchendurchmesser) < 2 μm mit praktisch keinen Anteilen > 4 μm erzielt werden.

Die Pulverpräparate können tablettiert oder direkt als rieselfähige Pulver verwendet werden. Bei Verwendung kaltwasserlöslicher Schutzkolloide können die Präparate als Instant-Trinkgranulate verwendet werden.

In den nachfolgenden Beispielen wird die Durchführung des erfindungsgemäßen Verfahrens näher erläutert. Prozentgehalte beziehen sich auf das Gewicht.


Beispiel 1


450 g Fischöl wurden in 2400 g Isopropanol gemeinsam mit 20 g dl-α-Tocopherol und 50 g Ascorbylpalmitat bei 25°C suspendiert und bei Einstellung des Druckbegrenzungsventils (13) auf 30 bar mit 3600 g Isopropanol in Mischkammer (7) kontinuierlich gemischt. Bei einer Dosiergeswindigkeit von 2 l/h auf der Suspensionsseite und von 3 l/h auf der Lösungsmittelseite wurde in der Mischkammer (7) eine Mischungstemperatur von 190°C eingestellt. Nach einer Verweilzeit von 0,3 Sekunden wurde die entstandene molekulardisperse Lösung in Mischkammer (11) mit iner auf pH 9,5 eingestellten Lösung von 250 g Gelatine und 50 g Saccharose in 39,7 l Wasser bei einer Durchsatzgeswindigkeit von 27 l/h gemischt. Man erhielt ein Zweiphasengemisch, in dem die Ölphase in Form submikroskopisch kleiner Teilchen vorlag. Die Teilchengrößenanalyse mittels Photonen-Korrelations-Spektroskopie (B. Chu, Laser Light Scattering; Academic Press, New York, 1974) lieferte für die Ölphase eine mittlere Teilchengröße von 0,26 μm bei einer Verteilungsbreite von ± 46 %.

Nach Abtrennen des Lösungsmittels unter vermindertem Druck bei 50°C in einer Destillationsapparatur wurde eine viskose Flüssigkeit erhalten, die durch Sprühtrocknung in ein stabiles, rieselfähiges, leicht wasserlösliches Trockenpulver überführt wurde. Der Ölgehalt des Präparates betrug 55 %. Nach Wiederauflösung des Trockenpulvers in Wasser wurde erneut ein Zweiphasengemisch erhalten, in dem die Ölphase mikrodispers bei einer mittleren Teilchengröße von 0,26 μm vorlag, ohne daß gleichzeitig Ölteilchen von über 1 μm vorhanden waren. Die fischartige Geruchs- und Geschmacksnote war weitgehend maskiert.


Beispiel 2


Entsprechend der Arbeitsweise von Bsp. 1, jedoch unter Zusatz von 100 g Orangenöl und 2 g Provitamin A (β-Carotin) zur Suspensionsphase, wurde ein Zweiphasengemisch erhalten, welches durch Sprühtrocknung in eine gelbes Trockenpulver überführt wurde, das durch ein angenehmes Orangenaroma gekennzeichnet war. Der Gesamtölgehalt des Präparates betrug 60 %.

Nach Auflösen in Wasser wurde eine orangefarbene Flüssigkeit erhalten, die durch ein angenehmes Orangenaroma und völlige Maskierung der fischartigen Geruchs- und Geschmacksnote gekennzeichnet war.


Beispiele 3 bis 5


Entsprechend der Arbeitsweise wie in den Bspn. 1 und 2 wurden bei Variation der Einsatzoffmengen Produkte erhalten, deren Feinverteilungsgrad in Tab. 1 charakterisiert ist.

Tabelle 1

| Bsp. | Fischöl (g) | Orangenöl (g) | Gelatine (g) | Teilchengröße (μm) | |
|---|---|---|---|---|---|
| | | | | Austrag Mischkammer (11) | Hydrosol des durch Sprühtrocknung erhaltenen Pulvers |
| 3 | 300 | - | 300 | 0,215 | 0,25 |
| 4 | 400 | 50 | 250 | 0,26 | 0,35 |
| 5 | 350 | 100 | 250 | 0,25 | 0,28 |

Beispiel 6

Wie in Beispiel 2, jedoch unter Verwendung von 250 g Gummiarabicum als Schutzkolloid wurde ein Zweiphasengemisch erhalten, in dem die Ölphase mikrodispers mit einer mittleren Teilchengröße von 270 nm ± 46 % vorlag. Das Produkt wurde durch Sprühtrocknung in ein kaltwasserlösliches Trockenpulver überführt.

Beispiel 7

Wie in Beispiel 2, jedoch unter Verwendung einer auf pH 8 eingestellten wäßrigen Lösung von 300 g Casein in 39,7 g Wasser wurde ein Zweiphasengemisch erhalten, in den die Ölphase mikrodispers mit einer mittleren Teilchengröße von 309 nm ± 44 % vorlag. Durch Zugabe von 210 ml 0,5-molarer Schwefelsäure wurde bei pH 4,6 ein Copräzipitat erhalten, das die mikrodisperse Ölphase in einer festen Caseinmatrix enthielt. Durch Abfiltrieren und anschließende Trocknung wurde ein Trockenpulver mit einem Gesamt-Ölgehalt von 60 % erhalten.

Beispiele 8 und 9

Analog Beispiel 2, jedoch unter Verwendung von Aceton bzw. Ethanol, wurden Zweiphasengemische erhalten, die durch mittlere Teilchengröße der Ölphase gem. Tabelle 2 gekennzeichnet waren.

Tabelle 2

| | Lösungsmittel | Mittlere Teilchengröße der Ölphase (nm) | |
|---|---|---|---|
| | | Zweiphasengemisch | Hydrosol |
| Bsp. 8 | Aceton | 306 ± 44 % | 292 ± 52 % |
| Bsp. 9 | Ethanol | 235 ± 49 % | 260 ± 48 % |

Beispiel 10

Eine Mischung von 450 g Fischöl, 50 g Ascorbylpalmitat, 100 g Orangenöl und 20 g α-Tocopherol wurden mit einer Lösung von 250 g Gelatine und 50 g Saccharin in 1840 g Wasser (nach Einstellung der wäßrigen Lösung auf pH 9,1 mittels 170 ml 1 n NaOH) mittels Ultra-Turrax® in eine grobteilige o/w-Emulsion überführt (Teilchengröße 1 bis 50 μm). Die Mischung wurde unter Verwendung eines Microfluidizers (M-100) der Fa. Biotechnology Development Corp. Newton, Mass., USA, homogenisiert. Die erhaltene o/w-Emulsion war durch eine mittlere Teilchengröße der Ölphase von 0,8 μm ± 34 % gekennzeichnet. Durch Sprühtrocknung wurde eine leichtlösliches Trockenpulver erhalten, das nach Auflösen in Wasser ein mikrodisperses Hydrosol (mittlere Teilchengröße ebenfalls 0,8 μm) mit angenehm aromatischer Geruchsnote lieferte.

Beispiel 11

Analog Beispiel 1 wurden 450 g Fischöl in 2400 g Isopropanol gemeinsam mit 20 g dl-α-Tocopherol, 50 g Ascorbylpalmitat und 100 g Orangenöl bei 180°C gelöst und in Mischkammer (11) mit 40 l einer auf pH 6,8 eingestellten wäßrigen Lösung von 92 g Casein und 206 g Lactose gemischt. Die Teilchengrößenanalyse des erhaltenen Zweiphasengemisches lieferte für den mittleren Durchmesser der mikrodispersen Ölphase 0,27 μm ± 2 %. Durch Sprühtrocknen wurde ein Trockenpulver erhalten, das in kaltem Wasser unter Rückbildung der mikrodispersen Ölphase gut löslich war. Die Lösung war durch eine angenehm aromatische Geruchsnote gekennzeichnet.

Beispiel 12

Analog Beispiel 10 wurden 450 g Fischöl und 42 g Ascorbylpalmitat mit einer Lösung von 290 g Lactose und 58 g Casein in 1960 g Wasser, die auf pH 6,7 eingestellt war, mittels Ultra Turrax® zu einer grobteiligen Emulsion voremulgiert (Teilchengröße 1 bis 50 μm) und anschließend unter Verwendung des Microfluidizers von Beispiel 10 zu einer o/w-Emulsion homogenisiert. Nach Sprühtrocknung der so erhaltenen Emulsion wurde ein kaltwasserlösliches Trockenpulver erhalt, das nach Wiederauflösen in Wasser eine o/w-Emulsion (Hydrosol) lieferte mit einer mittleren Teilchengröße von 0,9 μm ± 50 %.

## Patentansprüche

1. Verfahren zur Herstellung von mikrodispersen, pulverförmigen oder wäßrigen Fischölpräparaten, dadurch gekennzeichnet, daß man ein Fischöl gemeinsam mit einem Tensid, einem Schutzkolloid, Wasser und gegebenenfalls üblichen galenischen Hilfsstofen zunächst in einem herkömmlichen Schnellrührer voremulgiert und dann in einem Hochdruckhomogenisator bei einer Temperatur zwischen 0 und 50°C so fein emulgiert, daß der mittlere Durchmesser der Öltröpfchen unter 10 μm liegt, und das erhaltene Zweiphasengemisch gegebenenfalls durch Entzug von Wasser in ein Trockenpulver überführt.

2. Verfahren zur Herstellung von mikrodispersen, pulverförmigen oder wäßrigen Fischölpräparaten, dadurch gekennzeichnet, daß man ein Fischöl in einem flüchtigen, mit Wasser mischbaren organischen Lösungsmittel gemeinsam mit einem Tensid und ggf. einem Oxidationsstabilisator bei Temperaturen zwischen 10°C und 200°C löst, die erhaltene molekulardisperse Lösung durch Mischen mit einer wäßrigen Lösung eines Schutzkolloids, die übliche galenische Hilfsstoffe enthalten kann, bei Temperaturen zwischen 0°C und 50°C in eine Zweiphasenmischung zerlegt, wobei die Ölphase in Form submikroskopisch kleiner Teilchen bei einer mittleren Teilchengröße von weniger als 1 μm entsteht, das erhaltene Zweiphasengemisch in an sich bekannter Weise von dem Lösungsmittel befreit und ggf. durch gleichzeitigen oder nachfolgenden Entzug von Wasser in ein Trockenpulver überführt.

3. Verfahren zur Herstellung von mikrodispersen, pulverförmigen oder flüssigen Fischölpräparaten gemäß Anspruch 2, dadurch gekennzeichnet, daß man als wassermischbare flüchtige Lösungsmittel Alkohole, Ketone, Ester, Acetale oder Ether einsetzt, die zu mindestens 10 % wassermischbar sind, unter 200°C sieden und weniger als 7 Kohlenstoffatome enthalten.

4. Verfahren zur Herstellung von Fischölpräparaten gemäß Anspruch 3, dadurch gekennzeichnet, daß man als wassermischbares flüchtiges Lösungsmittel mindestens eines aus folgender Gruppe einsetzt: Aceton, Methanol, Ethanol, o-Propanol, Isopropanol, 1,2-Butandiol-1-methylether, 1,2-Propandiol-1-n-propylether, Tetrahydrofuran, Dioxan, Ethylmethylether und Methylacetat.

5. Verfahren zur Herstellung von Fischölpräparaten gemäß Anspruch 1 oder 2, dadurch gekennzeichnet, daß man als Tensid eines oder mehrere aus folgender Gruppe einsetzt: Ascorbylfettsäureester, Fettsäuremono- oder -diglyceride und deren Oxethylierungsprodukte, Ester von Monofettsäureglyceriden mit Essigsäure, Zitronensäure, Milchsäure oder Diacetylweinsäure, Polyglycerin-Fettsäurester, Sorbitanfettsäureester, Propylenglykolfettsäureester, 2-(2'-stearoyllactyl)-milchsaure Salze oder Lecithin.

6. Verfahren gemäß einem der Ansprüche 1 bis 5, dadurch gekennzeichnet, daß als Tensid Ascorbylpalmitat eingesetzt wird.

7. Verfahren gemäß einem der Ansprüche 2 bis 6, dadurch gekennzeichnet, daß die Herstellung der molekulardispersen Fischöllösung und die Phasentrennung in eine mikrodisperse Ölphase und eine wäßrige kontinuierliche Phase kontinuierlich in zwei in Reihe geschalteten Mischkammern erfolgt.

8. Flüssiges oder pulverförmiges Fischölpräparat, hergestellt nach dem Verfahren des Anspruchs 1 oder 2, enthaltend 5 bis 70 Gew.-% eines Fischöls, 1 bis 30 Gew.% eines Tensids, 5 bis 50 Gew.% eines Schutzkolloids, 0 bis 70 % eines Weichmachers, alle Prozentangaben bezogen auf die Trockenmasse, sowie 0 bis 50 % an üblichen galenischen Hilfsstoffen, gekennzeichnet durch Freiheit von Chlorkohlenwasserstoffen, eine mittlere Teilchengröße des Fischöls von weniger als 10 $\mu$m im Falle der Herstellung nach dem Verfahren des Anspruchs 1, bzw. von weniger als 1 $\mu$m im Falle der Herstellung nach dem Verfahren des Anspruchs 2.

9. Pulverförmiges Fischölpräparat nach Anspruch 8, enthaltend ein kaltwasserlösliches Polypeptid oder Polysaccharid als Schutzkolloid.

10. Pulverförmiges Fischölpräparat nach Anspruch 8, enthaltend ein über den pH-Wert in seiner Wasserlöslichkeit steuerbares Schutzkolloid.

## Claims

1. A process for the preparation of a microdisperse, pulverulent or aqueous fish oil preparation, wherein a fish oil together with a surfactant, a protective colloid, and, if required, conventional pharmaceutical auxiliaries are first pre-emulsified in a conventional high speed stirrer and then emulsified in a high pressure homogenizer at from 0 to 50°C so finely that the mean diameter of the oil droplets is less than 10 $\mu$m, and the resulting two-phase mixture is converted to a dry powder, if necessary by removal of water.

2. A process for the preparation of a microdisperse, pulverulent or aqueous fish oil preparation, wherein a fish oil is dissolved in a volatile, water-miscible organic solvent together with a surfactant and, if required, an antioxidant, at from 10 to 200°C, the resulting molecular disperse solution is divided into a two-phase mixture by mixing with an aqueous solution of a protective colloid, which may contain conventional pharmaceutical auxiliaries, at from 0 to 50°C, the oil phase being obtained in the form of submicroscopic particles having a mean particle size of less than 1 $\mu$m, and the resulting two-phase mixture is freed from the solvent in a conventional manner and converted to a dry powder, if necessary by simultaneous or subsequent removal of water.

3. A process for the preparation of a microdisperse, pulverulent or liquid fish oil preparation as claimed in claim 2, wherein the water-miscible volatile solvent used is an alcohol, a ketone, an ester, an acetal or an ether which is not less than 10% water-miscible, boils at below 200°C and contains less than 7 carbon atoms.

4. A process for the preparation of a fish oil preparation as claimed in claim 3, wherein one or more solvents from the group consisting of acetone, methanol, ethanol, n-propanol, isopropanol, butane-1,2-diol 1-methyl ether, propane-1,2-diol 1-n-propyl ether, tetrahydrofuran, dioxane, ethyl methyl ether and methyl acetate are used as the water-miscible volatile solvent.

5. A process for the preparation of a fish oil preparation as claimed in claim 1 or 2, wherein one or more surfactants from the group consisting of ascorbyl fatty acid esters, fatty acid mono- and diglyferides and their oxy-ethylation products, esters of mono-fatty acid glycerides with acetic acid, citric acid, lactic acid or diacetyltartaric acid, polyglycerol fatty acid esters, sorbitan fatty acid esters, propylene glycol fatty acid esters, 2-(2'-stearoyllactyl)-lactic acid salts and lecithin are used as the surfactant.

6. A process as claimed in any of claims 1 to 5, wherein the surfactant used is ascorbyl palmitate.

7. A process as claimed in any of claims 2 to 6, wherein the preparation of the molecular disperse fish oil solution and phase separation into a microdisperse oil phase and an aqueous continuous phase are carried out continuously in two mixing chambers connected in series.

8. A liquid or pulverulant fish oil preparation, prepared by the process of claim 1 or 2, containing from 5 to 70% by weight of a fish oil, from 1 to 30% by weight of a surfactant, from 5 to 50% by weight of a protective colloid, from 0 to 70% by weight of a plasticizer, all percentages being based on dry material, and from 0 to 50% by weight of conventional pharmaceutical auxiliaries, which preparation is free of chlorohydrocarbons and has a mean particle size of the fish oil of less than 10 $\mu$m when prepared by the process of claim 1 and of less than 1 $\mu$m when prepared by the process of claim 2.

9. A pulverulant fish oil preparation as claimed in claim 8, containing a polysaccharide or polypeptide which is soluble in cold water, as the protective colloid.

10. A pulverulant fish oil preparation as claimed in claim 8, containing a protective colloid whose water solubility can be controlled via the pH.

**Revendications**

1. Procédé de préparation de compositions d'huile de poissons microdispersées, pulvérulentes ou aqueuses, caractérisé en ce que l'on soumet d'abord une huile de poissons à émulsion préalable dans un agitateur rapide de type connu avec un agent tensioactif, un colloïde protecteur, de l'eau et le cas échéant des produits auxiliaires galéniques usuels puis on émulsionne finement dans un homogénéiseur à haute pression, à une température de 0 à 50 degrés C, jusqu'à ce que le diamètre moyen des gouttelettes d'huile soit inférieur à 10 $\mu$m et le cas échéant on convertit le mélange à deux phases ainsi obtenu en poudre sèche par élimination de l'eau.

2. Procédé de préparation de compositions d'huile de poissons microdispersées, pulvérulents ou aqueuses, caractérisé en ce que l'on dissout une huile de poissons dans un solvant organique volatil non miscible avec l'eau avec un agent tensioactif et le cas échéant un stabilisant contre l'oxydation à des températures de 10 à 200 degrés C, on convertit la solution moléculaire ainsi obtenue, par mélange avec une solution aqueuse d'un colloïde protecteur pouvant contenir des produits auxiliaires galéniques usuels, à des températures de 0 à 50 degrés C, en un mélange à deux phases dans lequel la phase huileuse est formée de particules submicroscopiques d'une dimension moyenne inférieure à 1 $\mu$m on élimine le solvant de manière connue en soi du mélange à deux phases et le cas échéant on convertit ce dernier en une poudre sèche par élimination simultanée ou consécutive de l'eau.

3. Procédé de préparation de compositions d'huile de poissons microdispersées, pulvérulentes ou liquides selon revendication 2, caractérisé en ce que l'on utilise en tant que solvants volatils miscibles avec l'eau des alcools, des cétones, des esters, des acétals ou des éthers miscibles avec l'eau en proportion d'au moins 10 %, bouillant au-dessous de 200 degrés C et contenant moins de 7 atomes de carbone.

4. Procédé de préparation de compositions d'huile de poissons selon revendication 3, caractérisé en ce que l'on utilise en tant que solvant volatil miscible avec l'eau au moins un solvant du groupe suivant : l'acétone, le méthanol, l'éthanol, le n-propanol, l'isopropanol, l'éther 1-méthylique du 1,2-butane-diol, l'éther 1-n-propylique du 1,2-propane-diol, le tétrahydrofuranne, le dioxanne, l'oxyde de méthyle et d'éthyle et l'acétate de méthyle.

5. Procédé de préparation de compositions d'huile de poissons selon revendication 1 ou 2, caractérisé en ce que l'on utilise en tant qu'agents tensioactifs un ou plusieurs agents tensioactifs du groupe suivant : les esters ascorbyliques d'acides gras, les mono- ou diglycérides d'acides gras et leurs produits d'oxyéthylation, les esters de monoglycérides d'acides gras et de l'acide acétique, de l'acide citrique, de l'acide lactique ou de l'acide diacétyltartrique, les esters d'acides gras et de polyglycérol, les esters d'acides gras et de sorbitanne, les esters d'acides gras et de propylène-glycol, les sels de l'acide 2-(2'-stéaroyllactyl)-lactique ou de lécithine.

**6.** Procédé selon une des revendications 1 à 5, caractérisé en ce que l'on utilise en tant qu'agent tensioactif le palmitate d'ascorbyle.

**7.** Procédé selon l'une des revendications 2 à 5, caractérisé en ce que la préparation de la solution moléculaire d'huile de poissons et la séparation des phases en une phase huileuse microdispersée et une phase aqueuse continue sont réalisées en continu dans deux chambres de mélange disposées en série.

**8.** Composition d'huile de poissons liquide ou pulvérulente préparée par le procédé de la revendication 1 ou 2, contenant 5 à 70 % en poids d'une huile de poissons, 1 à 30 % en poids d'un agent tensioactif, 5 à 50 % en poids d'un colloïde protecteur, 0 à 70 % en poids d'un plastifiant, toutes ces indications de pourcentage s'entendant en matières sèches, et 0 à 50 % de produits auxiliaires galéniques usuels, caractérisée en ce qu'elle est exempte d'hydrocarbures chlorés, et en ce que la dimension de particule moyenne de l'huile de poissons est inférieure à 10 $\mu$m dans le cas de la préparation par le procédé de la revendication 1 et inférieure à 1 $\mu$m dans le cas de la préparation par le procédé de la revendication 2.

**9.** Composition d'huile de poissons pulvérulente selon la revendication 8, contenant un polypeptide soluble dans l'eau froide ou un polysaccharide en tant que colloïde protecteur.

**10.** Composition d'huile de poissons pulvérulente selon revendication 8, contenant un colloïde protecteur dont la solubilité dans l'eau peut être contrôlée par le pH.

I    II    III